(19) Europäisches Patentamt

European Patent Office

Office européen des brevets

(11) **EP 2 512 341 B1**

(12) **EUROPEAN PATENT SPECIFICATION**

(45) Date of publication and mention
of the grant of the patent:
**04.06.2014 Bulletin 2014/23**

(21) Application number: **09798965.1**

(22) Date of filing: **16.12.2009**

(51) Int Cl.:
***A61B 6/00*** *(2006.01)*

(86) International application number:
**PCT/IB2009/055791**

(87) International publication number:
**WO 2011/073728 (23.06.2011 Gazette 2011/25)**

(54) **BONE MINERAL DENSITY CHANGE RATE IDENTIFICATION**

ERKENNUNG DER KNOCHENMINERALDICHTEVERÄNDERUNGSRATE

IDENTIFICATION DE LA VITESSE DE VARIATION DE LA DENSITÉ MINÉRALE OSSEUSE

(84) Designated Contracting States:
**AT BE BG CH CY CZ DE DK EE ES FI FR GB GR
HR HU IE IS IT LI LT LU LV MC MK MT NL NO PL
PT RO SE SI SK SM TR**

(43) Date of publication of application:
**24.10.2012 Bulletin 2012/43**

(73) Proprietor: **Sectra Imaging IT Solutions AB
583 30 Linköping (SE)**

(72) Inventors:
• **KÄLVESTEN, Johan
S-58214 Linköping (SE)**

• **ALGULIN, Jakob
S-58937 Linköping (SE)**

(74) Representative: **Plougmann & Vingtoft A/S
Rued Langgaards Vej 8
2300 Copenhagen S (DK)**

(56) References cited:
**WO-A2-2004/112580      DE-A1- 3 726 456
US-A- 5 005 196      US-B1- 6 711 282**

**Description**

FIELD OF THE INVENTION

The present invention relates inter alia to a method of identifying the change rate in bone mineral density (BMD) over time and to an apparatus for carrying out such method.

BACKGROUND OF THE INVENTION

For the purpose of the disclosure below, it is mentioned that bone mineral density typically is defined as the density mass of "bone mineral" and that the BMD as well as the change rate does not provide any diagnosis. In particular, further evaluation including other parameters must be performed to provide a diagnosis.

It is known that BMD in the bones of the hand is dramatically reduced in the early phases of the autoimmune disorder rheumatoid arthritis (RA). It has recently been shown in several studies that a sharp decline in BMD is highly predictive of a negative disease outcome. Since there is a lack of good predictors for long term outcome in RA, the change rate of BMD in the bones of the hand is of high clinical relevance to support the physician in treatment decisions.

BMD is often determined by image processing of an x-ray image, which image processing involves the image being divided into pixels. This image processing results in numerous problems to be solved.

In relation to the present invention, one of the problems pertaining to determining the change rate of BMD by measuring the BMD twice, within a reasonable separation in time, is that the pixel resolution of the measurements by which BMD is determined from is generally not high enough to capture the small changes that occur in the bones within reasonable time frame. Thus this pixel resolution demands an increase of the reasonable time frame interval between the BMD measurements so as to obtain detectable differences in the measurements by which BMD is determined from.

Among the techniques used for measuring BMD, the determination of BMD based on digital x-ray radiogrammetry, resides in the evaluation of the thickness and width of the cortical bone of interest. Determining these parameters from for example a digitized X-ray image acquired with the maximum resolution offered by today's standard medical X-ray machines, is hard to do because of the low resolution of the images compared to the change in thickness and width of the cortical bone of interest.

EP 1046374B1 discloses an apparatus for estimating BMD by the use of radiogrammetry. The disclosed apparatus comprises of means to obtain two-dimensional image data relating to the cortical bone. Further, it comprises computer means for determining the cortical thickness (t) and the width (w) of the cortical bone of interest and estimating the BMD by said computer means as; $BMD = const._B \times t \times (1 - \frac{t}{w})$, where const.$_B$ is determined from a calibration and pairs of (t, w) have been calibrated to corresponding BMD values for one or more bones.

The inventors have realized that due to the limited resolution offered by today's X-ray machines a clinical relevant change in thickness and width of the bones can be detected only if these parameters are evaluated after a long period of time. Therefore over short period of time a valuable determination of BMD change rate can not be performed in particular for diseases, such as RA which demands high resolution as the change in thickness and width of the bone is smaller than the resolution of digitized X-rays images acquired over a typical examination period.

Being able to accurately estimate change rate of BMD over time of a cortical bone has been shown to have clinical value in for instance in several diseases, e.g. RA and is contemplated to be clinically significant in some other autoimmune disorders as well. Further BMD is used together with other known risk factors to diagnose possible diseases, e.g. osteoporosis and assess fracture risk where BMD is a relevant indicator.

The inventors of the present invention have appreciated that an improved method of identifying the change rate of bone mineral density over time is of benefit, and has in consequence devised the present invention.

SUMMARY OF THE INVENTION

It is an object of the invention to provide a method of improving the BMD change rate determination from X-ray images taken within a typical clinical disease specified time interval. According to the invention, determination of the width and the thickness of a cortical bone of interest are used as the basis for the calculation of the BMD performed at two time points separate from each other. In the present invention, calculation of BMD is performed with the help of weights associated to the thickness and width of the cortical bone of interest.

This invention is particularly, but not exclusively, advantageous as the time frame interval needed to obtain a detectable difference in the measurement of BMD can be reduced thus improving the clinical value of the BMD evaluation.

Thus the above described object and several other objects are intended to be obtained in a first aspect of the invention by providing a method for bone mineral density change rate identification based on at least two images of the same at least one bone taken at two different points in time and the method comprising: determining internal and/or external edge location of the at least one bone from the at least two images; calculating a bone mineral density change rate (BMDCR) by using at least one weighting factor for the variation of the internal and/or external edge location, wherein the significance of said at least one weighting factor is selected based on the cause of the bone mineral change.

Preferably, the invention does not include the step of taking or providing the images.

Bone mineral density (BMD) is herein defined as the mass of "bone mineral" per square cm, typically of a specific bone area on a radiograph. The unit used is typically $g/cm^2$ or $mg/cm^2$. BMD may be also referred as bone mineral per volume or bone mineral per distance of bone. In this case the method can be applied with the necessary variations in the calculation of the BMD having been made.

"Change" is herein defined as different in value and/or structure and is herein referred also as "variation".

In some embodiments images of the same at least one bone may be digitized, e.g. put into a digital form by scanning a printed image. In some other embodiments images of the same at least one bone may be digital, e.g. acquired directly by a digital detector. In some embodiments these images may be digital X-ray images.

In some embodiments the at least two images comprise the 2nd, 3rd and 4th metacarpals. The images of 2nd, 3rd and 4th metacarpals are generally for the identification of BMDCR in RA.

Several technologies may be used for estimating BMD, such as digital X-ray radiogrammetry (DXR), magnetic resonance tomography (MRI), quantitative computer tomography (QCT), dual X-ray absorptiometry (DEXA), Radiographic absorptiometry (RA), quantitative ultrasound (QUS). When DXR is used to estimate BMD, the resulting BMD value is generally referred to as DXR-BMD.

In order to determine the change rate of BMD a discretization of $\partial BMD/\partial t$ is applied e.g. as $[BMD(t_2)-BMD(t_1)]/[t_2-t_1]$ whereby the rate of change in principle should be determinable by determining BMD at two different points in time.

Two different points in time may be defined as distant between each other for a clinically meaningful period of time and therefore depending on the type of disease. For example, when the disease is RA, such a period between the two different points in time may be less than two years, preferably less than 1 year, more preferably less than 6 months, even more preferably less than 3 months.

In a specific preferred embodiment, BMD is used to determine the rate of change (BMDCR). In such embodiment, BMD is calculated based on a functional relationship between thickness and width of the cortical bone, (see for instance EP 1046374). Accordingly, the function could preferably have the following appearance:

$$BMD = const._B \times T \times (1 - \frac{T}{W})$$

where const.$_B$ is determined from a calibration and pairs of (T, W) have been calibrated to corresponding BMD values for one or more bones.

In some embodiments according to the first aspect of the invention the method further comprises: determining one or more parameters related to bone mineral density; calculating a bone mineral density change rate (BMDCR) also by using at least one weighting factor for the variation of the one or more parameters related to bone mineral density, wherein the significance of said at least one weighting factor is selected based on the cause of the bone mineral density change.

For example a parameter related to bone mineral density may be porosity (P) of the bone.

The method for the identification of the bone mineral density change rate comprises a calculation that takes into account the variation over the parameters used to calculate BMD, e.g. the location of the internal and/or external edge of the cortical bone, and/or the changes over the properties which influence the BMD, e.g. porosity of the bone. In doing this the calculation comprised in the method of the present invention includes the use of weighting factors for the variations of these parameters and properties.

The weighting factors express the influence that one property e.g. thickness, width or porosity has on the determination of the rate of change, i.e. weighting factors in the calculation of the BMDCR are used to weight the importance of the variations between the two points in time of the parameters, e.g. edge locations and properties, e.g. porosity, which influence the calculation of the BMD.

Different significance, i.e. different weight, is therefore given to the weighting factors of the different parameters and properties depending on the cause of the variation in bone mineral density.

For example in some embodiments the bone mineral density variation may be induced by a disease. In some other embodiments the disease inducing the bone mineral density variation is rheumatoid arthritis (RA). In some other embodiments the disease inducing the bone mineral density variation is psoriatic arthritis (PsA).

In the calculation of the BMDCR different significance of weighting factors for the same parameters or properties influencing the calculation of the BMD may be used for different disease induced bone mineral density variation.

For example if for BMD changes induced by RA the change rate of the location of the internal edge is larger than the change rate of the location of the external edge of the bone in relation to the accuracy with which those respective edge locations can be measured, the weighting factor should ponder this difference, i.e. the weighting factor for the location of the internal edge is higher in value than the one for the location of the external edge. For bone mineral density changes induced by PsA, the significance of the weighting factors may be inverted so that the relative change rate of the location of the internal edge is smaller than the relative change rate of the location of the external edge of the bone, i.e. the weighting factor for the location of the internal edge is lower in value than the one for the location of the external edge.

In some embodiments the method according to the first aspect of the invention comprises: determining the endosteal edge location (EEL), periosteal edge location (PEL) of the at least one bone from said at least two images; calculating a bone mineral density change rate (BMDCR) by using at least one weighting factor for the change in the PEL which is lower than or equal to the at least one weighting factor for the change of EEL.

In some embodiments the method according to the first aspect of the invention comprises: determining the endosteal edge location (EEL), periosteal edge location (PEL) and porosity (P) of the at least one bone from the at least two images; calculating a bone mineral density change rate (BMDCR) by using at least one weighting factor for the change in the PEL which is lower than or equal to the at least one weighting factor for the change of P and is lower than the at least one weighting factor for the EEL.

The locations of the Endosteal edge (EEL) and of the Periosteal edge (PEL) of the cortical bone are used to define thickness and width of the bones to be measured.

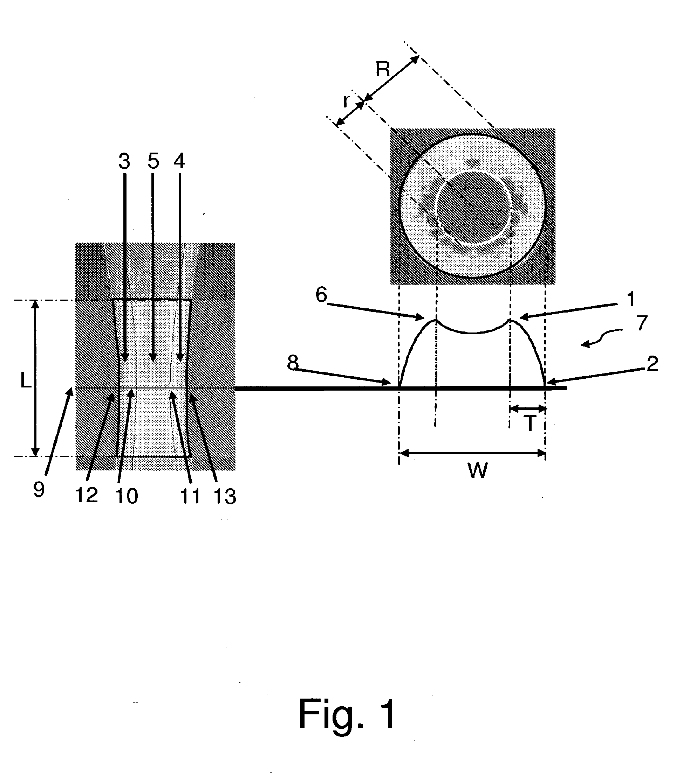

Thickness and width are clearly defined in figure 1 and in the following section regarding the description of the embodiments.

The method for the identification of the bone mineral change rate comprises the calculation of the BMDCR that takes into account the differences between parameters like EEL, PEL and P at the two different points in time.

In some embodiments the calculation takes into account the change in properties of the bones which can be evaluated from differences in EEL, PEL and P, e.g. differences in the distance or in the area between the EEL.

In some other embodiments the calculation takes into account the differences in other properties of the bones which can be deducted from the parameters like EEL, PEL and P, e.g. the differences at the two points in time in the volume of the bones analysed.

The method for the identification of the bone mineral density change rate comprising the calculation of BMDCR comprises the use of a weighting factor for the estimation of the BMDCR.

The weighting factors express the influence that one property e.g. thickness, width or porosity has on the determination of the rate of changing, i.e. weighting factors in the calculation of the BMDCR are used to weight the importance of the variations between the two points in time when the images are acquired in the parameters, e.g. edge locations and properties, e.g. porosity, which influence the calculation of the BMD.

In order to improve the accuracy in the estimation of BMDCR, the calculation is improved by taking into account that the weighting factor should ponder also that some properties statistically change more and some other change less relative to the precision with which they can be measured.

For example the relative change rate of the properties of thickness and width and porosity may be different:

$$\frac{CR_{PEL}}{\sigma_{PEL}} \neq \frac{CR_{EEL}}{\sigma_{EEL}} \neq \frac{CR_P}{\sigma_P}$$

Where CR is the change rate and σ is the magnitude of the standard error for PEL, EEL and P respectively.

For example, the relative change rate for the EEL may be larger than the one for P and even larger than the one for PEL:

$$\frac{CR_{EEL}}{\sigma_{EEL}} > \frac{CR_P}{\sigma_P} > \frac{CR_{PEL}}{\sigma_{PEL}}$$

This means that the true change in PEL between two measurements in two points in time may be small and contribute less to the total true change in bone mineral density (BMD); while at the same time may cause a significant amount of noise in the BMD measurement. Therefore, the accuracy of the relative change rate estimation can thus be improved by making a measured change in one or more of EEL, PEL and P contribute less to the BMD change rate identifier.

If more weight is given to the change in those properties that statistically change more relative to their measurement precision, it means that less weight is given to the change in properties that statistically have a smaller true underlying change relative to the precision with which they can be measured.

Therefore where the relative change rate for the EEL may be larger than the one for P and even larger than the one for PEL, the calculation of the BMDCR by using a weighting factor for the change in the PEL which is lower than or equal to the weighting factor for the change of P and is lower than the weighting factor for the EEL provides a better estimation of the BMDCR when the change is caused by RA.The previously described objects and several other objects are intended to be obtained in a second aspect by an apparatus adapted to perform the method according to the first aspect of the invention.

In some embodiments according to any aspect of the invention the internal and/or external edge location of the at least one bone from the at least two images are determined for a plurality of individual lines extending in the direction perpendicular to the longitudinal direction of the bone and being positioned at different positions along the longitudinal direction of the bone, and wherein the calculation of the BMDCR is determined on the basis of pairs of the internal and/or external edge location of the at least one bone from the at least two images corresponding to the individual lines.

In some other embodiments according to one embodiment of the invention the internal and/or external edge location of the at least one bone from the at least two images are determined on the basis of their mean location for each of the individual lines.

Preferably values from the internal and/or external edge location of the at least one bone from the at least two images are determined on the basis of their mean location for each of more than 10 lines, such as more than 40 lines, such as more than 60 lines, such as more than 80 lines, such as more than 100 lines per cm of the bone within a predetermined longitudinal part of the bone.

In general the various aspects of the invention may be combined and coupled in any way possible within the scope of the invention. These and other aspects, features and/or advantages of the invention will be apparent from and elucidated with reference to the embodiments described hereinafter.

BRIEF DESCRIPTION OF THE DRAWINGS

Embodiments of the invention will be described, by way of example only, with reference to the drawing, in which figure 1 shows a digital image of a front of a bone, a bone density profile and an estimate of the cross section of a bone based on the bone density profile.

DESCRIPTION OF EMBODIMENTS

Figure 1 shows a digital image of a front of a bone, a bone density profile and an estimate of the cross section of a bone based on the bone density profile, where the parameters necessary for the calculation of the BMDCR are shown.

In a preferred embodiment the calculation of BMDCR involves a radiogrammetric and textural analysis of the three middle metacarpal bones of a hand.

In a preferred embodiment, the location of the bones in the radiograph is found by a model-based algorithm known as the Active Shape Model (ASM). The ASM algorithm has been adapted to find the diaphysis of the three middle metacarpals in the hand. After each diaphysis has been identified, regions of interest (ROI) are placed automatically for the three metacarpals.

The algorithm places the three ROI's in a coupled fashion by sliding them in a partly fixed configuration along the bone shafts to a position identified by the minimum combined bone width 9. The heights of the ROIs are fixed to 2.0 cm, 1.8 cm, and 1.6 cm for the 2nd, 3rd, and 4th metacarpals, respectively.

Within each ROI, the endosteal (inner) edges 10 and 11, and periosteal (outer) edges 12 and 13 are automatically found, thereby segmenting the bone into two cortical regions 3 and 4 and one endosteal region 5. Along a profile 7 across the bone, the two endosteal edge points 10 and 11 are associated with the points of maximal intensity 6 and 1 as illustrated in Figure 1.

The periosteal edge points 12 and 13 are associated with the points of maximal curvature 8 and 2 in the bone profile. The average cortical thickness ($T_i$), and bone width ($W_i$) are determined for each metacarpal accumulating over 90-204 measurements per centimeter bone for resolutions between 230 dpi and 519 dpi. This for example implies that a total of 300-700 measurements contribute to the average cortical thickness of a single bone.

A bone volume per projected area (VPA, measured in cm) is computed for each metacarpal bone assuming a cylindrically-shaped bone:

$$VPA_i = \pi \times \frac{\left(R_i^2 - r_i^2\right)}{W_i} = \pi \times T_i \times \left(1 - \frac{T_i}{W_i}\right),$$

where $R_i = \frac{W_i}{2}$, is the radius of the bone, and $r_i = \frac{W_i - 2 \times T_i}{2}$ is the radius of the medullary cavity, between the endosteal edges. These parameters are related to the PEL, EEL. For example the width $W_i$ is calculated as the difference between PEL 2 and 8 of the bone, while $T_i$ is the average value of the two cortical thicknesses, i.e. the average between PEL 8 and EEL 6 and PEL 2 and EEL 1.

The total VPA for the metacarpals is defined as a weighted average:

$$VPA_{mc} = \frac{(VPA_2 + VPA_3 + 0.5 \times VPA_4)}{2.5},$$

where the index at the feet of VPA indicates which metacarpal bone is referred to, e.g. $VPA_4$ refers to the fourth metacarpal bone. As it can be seen from the equation, to the fourth metacarpal bone is given a lower weight due to a lower precision and lower clinical importance.

DXR-BMD is then calculated as:

$$DXR - BMD = c \times VPA_{mc} \times (1 - P),$$

where the scaling constant c is determined so that DXR-BMD on the average is equal to that of the mid-distal forearm region of a densitometer, e.g. Hologic QDR 2000 densitometer (Hologic, Waltham, MA, USA). The constant adapts VPA to both the volumetric mineral density of compact bone and the typical shape characteristics of the involved bones. P is an estimated three-dimensional porosity, aimed to be the fraction of the cortical bone volume that is not occupied by bone.

The change rate of BMD is measured at two different points in time ($t_1$ and $t_2$) and the difference in their measured value is divided by the difference in time between the measurements:

$$\frac{d}{dt}BMD = \frac{BMD_{t2} - BMD_{t1}}{t_2 - t_1}.$$

Therefore the DXR-BMD change rate is composed of the measured values of Periosteal edge location (PEL), Endosteal edge location (EEL) and Porosity estimation (P) of each side of each of the metacarpal bones at the two different points in time. In other words:

$$\frac{d}{dt}BMD = f\left(EELt_1, EELt_2, PELt_1, PELt_2, Pt_1, Pt_2, t_2 - t_1\right).$$

Each of these three properties can be measured with a certain precision, e.g. the measured value of PEL at point $t_1$ is:

$$PEL = \overline{PEL}_{t1} + \varepsilon_{PEL} \; ,$$

where the magnitude of the standard error is denoted $\sigma_{PEL}$ for $\varepsilon_{PEL}$ and correspondingly for the other properties.

Any disease induced change in DXR-BMD, such as for example the disease induced change observed in RA, is due to a change in the PEL, EEL and P.

When estimating the change rate of BMD in a patient's bone, there is a trade off between the time between measurements and the achieved accuracy of the BMD change rate estimation:

$$\frac{d}{dt}BMD \approx \frac{BMD_{t2} - BMD_{t1}}{t_2 - t_1} = \frac{\overline{BMD}_{t2} + \varepsilon_{t2} - \overline{BMD}_{t1} + \varepsilon_{t1}}{t_2 - t_1} = \frac{d}{dt}\overline{BMD} + c\frac{\varepsilon_{BMD}}{t_2 - t_1} \; ,$$

thus the accuracy error is proportional to $\dfrac{\varepsilon_{BMD}}{t_2 - t_1}$, i.e. reducing the time between BMD measurements by a factor two, leads to approximately a doubling of the accuracy error of the BMD change rate estimation.

According to the invention the accuracy of the DXR-BMD change rate estimation is improved if more weight is given to the change in those properties that statistically change more relative to their measurement precision than if more weight is given to the change in properties that statistically have a smaller true underlying change relative to the precision with which they can be measured.

For example as it has been found for RA that the disease induced relative change rate in PEL, EEL and P, denoted DICRPEL, DICREEL, DICRP respectively over short time periods, it may be so that,

$$\frac{DICR_{EEL}}{\sigma_{EEL}} > \frac{DICR_P}{\sigma_P} > \frac{DICR_{PEL}}{\sigma_{PEL}} \, .$$

Thus for example, the true disease induced change in PEL between two measurements at two different points in time may be small and contribute less to the total true change in DXR-BMD, while at the same time causing a significant amount of noise in the DXR-BMD measurement. The accuracy of the relative change rate estimation can be improved by making a measured change in one or more of EEL, PEL and P to contribute less to the DXR-BMD change rate identifier through the use of weighting factors.

By using weighting factors in the calculation of BMDCR for how much a change in one property shall influence the change rate identifier it may be based on the relation between the true change probability density, measurement precision for the property and those values in relation to corresponding values for the other two properties.

For example, using weighting factors $w_{PEL}$, $w_{EEL}$ and $w_p$, the BMD change rate identifier may for example be

estimated by, $\dfrac{d}{dt}BMD = \dfrac{BMD_{t2} - BMD'_{t1}}{t_2 - t_1}$ where BMD'$_{t1}$ is deduced by replacing PEL$_{t1}$ with $PEL_{t2} + w_{PEL} \times$

($PEL_{t1}$ - $PEL_{t2}$) and in an analogous way with EEL and P.

In some embodiments the method according to the invention the at least one weighting factor for the change in the PEL is zero.

In some other embodiments the at least one weighting factor for the change of the P is zero.

In some embodiments the at least one weighting factor for the change of the EEL is one.

For example, it may be that in the calculation of BMDCR for RA, to $w_{PEL}$ and $w_p$ is assigned a zero value, while to $w_{EEL}$ is assigned one. In this case the estimation of BMDCR still depends on the absolute value of P and PEL at one of the points in time so that

$$\frac{d}{dt}BMD = f\left(EELt_1, EELt_2, PELt_2, Pt_2, t_2 - t_1\right)$$

As $PEL_{t1}$ and $P_{t1}$ are equal to $PEL_{t2}$ and $P_{t2}$ respectively, by replacing a weighting factor of zero in the equations:

$$PEL_{t1} = PEL_{t2} + w_{PEL} \times (PEL_{t1} - PEL_{t2}),$$

and

$$P_{t1} = P_{t2} + w_P \times (P_{t1} - P_{t2}).$$

In some other embodiments the different parameters may carry different significance. For example in other diseases the induced relative change rate in PEL, EEL and P may have different importance. For example in the calculation of BMDCR induced by PsA it may be so that:

$$\frac{DICR_{PEL}}{\sigma_{PEL}} > \frac{DICR_P}{\sigma_P} > \frac{DICR_{EEL}}{\sigma_{EEL}}.$$

In this case the true disease induced change in PEL between two measurements at two different points in time may be high and contribute more to the total true change in DXR-BMD. In this case in order to improve the accuracy of the relative change rate estimation the contribution of PEL and P to the DXR-BMD change rate identifier through the use of weighting factors, should be more than the one of EEL. Therefore by using weighting factors which depends upon the disease induced change in BMD, calculation of BMDCR can be improved for several types of bones diseases.

While the invention has been illustrated and described in detail in the drawing and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawing, the disclosure, and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program related to the method of the invention may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

An apparatus designed to perform the invention may consist of a computer with a computer program related to the method. Preferably the computer will have software and hardware means to receive images from hospitals and clinics and to send reports to users. Preferably data can be sent over a telecommunications system.

## Claims

1.  A method for bone mineral density change rate identification based on at least two images of the same at least one bone taken at two different points in time and the method comprising:

    - determining internal and/or external edge location of said at least one bone from said at least two images;
    - calculating a bone mineral density change rate (BMDCR) by using at least one weighting factor for the variation between the first and the second image of said internal and/or external edge location, wherein the significance of said at least one weighting factor is selected based on the cause of the bone mineral density change.

2. A method for bone mineral density change rate identification according to claim 1 the method further comprising:

   - determining one or more parameters related to bone mineral density,
   - calculating a bone mineral density change rate (BMDCR) also by using at least one weighting factor for the variation between the first and the second image of said one or more parameters related to bone mineral density, wherein the significance of said at least one weighting factor is selected based on the cause of the bone mineral density change.

3. A method for bone mineral density change rate identification according to claim 1, wherein said internal edge location is the endosteal edge location (EEL), and said external edge location is the periosteal edge location (PEL) of said at least one bone from said at least two images, and said calculating a bone mineral density change rate (BMDCR) uses at least one weighting factor for the change between the first and the second image in the PEL which is lower than or equal to the at least one weighting factor for the change between the first and the second image of EEL.

4. A method for bone mineral density change rate identification according to claim 2, wherein said internal edge location is the endosteal edge location (EEL), said external edge location is the periosteal edge location (PEL) and wherein said one or more parameters related to bone mineral density is the porosity (P) of said at least one bone from said at least two images, and said calculating a bone mineral density change rate (BMDCR) uses at least one weighting factor for the change between the first and the second image in the PEL which is lower than or equal to the at least one weighting factor for the change between the first and the second image of P and is lower than the at least one weighting factor for the EEL.

5. A method according to claim 3 and 4 wherein the at least one weighting factor for the change between the first and the second image in the PEL is zero.

6. A method according to the preceding claims 3-5, wherein the at least one weighting factor for the change between the first and the second image of the P is zero.

7. A method according to the preceding 3-6 claims wherein the at least one weighting factor for the change between the first and the second image of the EEL is one.

8. A method according to any of the preceding claims wherein said at least two images comprise the 2nd, 3rd and 4th metacarpal bones.

9. A method according to any of the preceding claims, wherein said internal and/or external edge location of said at least one bone from said at least two images are determined for a plurality of individual lines extending in the direction perpendicular to the longitudinal direction of the bone and being positioned at different positions along the longitudinal direction of the bone, and wherein the calculation of the BMDCR is determined on the basis of pairs of said internal and/or external edge location of said at least one bone from said at least two images corresponding to the individual lines.

10. A method according to claim 9 wherein said internal and/or external edge location of said at least one bone from said at least two images are determined on the basis of their mean location for each of the individual lines.

11. A method according to claim 9 wherein said internal and/or external edge location of said at least one bone from said at least two images are determined on the basis of their mean location for each of more than 10 lines per cm of the bone within a predetermined longitudinal part of the bone.

12. A method according to claim 9 wherein said internal and/or external edge location of said at least one bone from said at least two images are determined on the basis of their mean location for each of more than 40 lines per cm of the bone within a predetermined longitudinal part of the bone.

13. A method according to claim 9 wherein said internal and/or external edge location of said at least one bone from said at least two images are determined on the basis of their mean location for each of more than 60 lines per cm of the bone within a predetermined longitudinal part of the bone.

14. A method according to claim 9 wherein said internal and/or external edge location of said at least one bone from said at least two images are determined on the basis of their mean location for each of more than 80 lines per cm of the bone within a predetermined longitudinal part of the bone.

15. An apparatus comprising hardware means and software means adapted to perform the method according to any of the preceding claims.

**Patentansprüche**

1. Verfahrenen zur Bestimmung der Knochenmineraldichteänderungsrate auf der Grundlage von mindestens zwei Bildern desselben mindestens einen Knochens, die zu zwei verschiedenen Zeitpunkten gemacht wurden, wobei das Verfahren umfasst:

    - Bestimmen der Lage des Innen-und/oder Außenrands des mindestens einen Knochens anhand der mindestens zwei Bilder;
    - Berechnen einer Knochenmineraldichteänderungsrate (BMDCR) unter Verwendung mindestens eines Gewichtungsfaktors für die Abweichung zwischen dem ersten und dem zweiten Bild der Lage des Innen-und/oder Außenrands, wobei die Bedeutung des mindestens einen Gewichtungsfaktors auf der Grundlage der Ursache der Knochenmineraldichteänderung ausgewählt wird.

2. Verfahren zur Bestimmung der Knochenmineraldichteänderungsrate nach Anspruch 1, wobei das Verfahren weiterhin umfasst:

    - Bestimmen eines oder mehrerer Parameter, die mit der Knochenmineraldichte in Zusammenhang stehen,
    - Berechnen einer Knochenmineraldichteänderungsrate (BMDCR) ebenfalls unter Verwendung mindestens eines Gewichtungsfaktors für die Abweichung zwischen dem ersten und dem zweiten Bild des einen oder der mehreren Parameter, die mit der Knochenmineraldichte in Zusammenhang stehen, wobei die Bedeutung des mindestens einen Gewichtungsfaktors auf der Grundlage der Ursache der Knochenmineraldichteänderung ausgewählt wird.

3. Verfahren zur Bestimmung der Knochenmineraldichteänderungsrate nach Anspruch 1, wobei die Lage des Innenrands die Lage des endostalen Rands (EEL) und die Lage des Außenrands die Lage des periostalen Rands (PEL) des mindestens einen Knochens auf den mindestens zwei Bildern ist und wobei das Berechnen einer Knochenmineraldichteänderungsrate (BMDCR) mindestens einen Gewichtungsfaktor für die Änderung der PEL zwischen dem ersten und dem zweiten Bild verwendet, die kleiner oder gleich dem mindestens einen Gewichtungsfaktor für die Änderung der EEL zwischen dem ersten und dem zweiten Bild ist.

4. Verfahren zur Bestimmung der Knochenmineraldichteänderungsrate nach Anspruch 2, wobei die Lage des Innenrands die Lage des endostalen Rands (EEL) und die Lage des Außenrands die Lage des periostalen Rands (PEL) ist und wobei der eine oder die mehreren Parameter, die mit der Knochenmineraldichte in Zusammenhang stehen, die Porosität (P) des mindestens einen Knochens auf den mindestens zwei Bildern ist und wobei das Berechnen einer Knochenmineraldichteänderungsrate (BMDCR) mindestens einen Gewichtungsfaktor für die Änderung der PEL zwischen dem ersten und dem zweiten Bild verwendet, die kleiner oder gleich dem mindestens einen Gewichtungsfaktor für die Änderung von P zwischen dem ersten und dem zweiten Bild sowie kleiner als der mindestens eine Gewichtungsfaktor für EEL ist.

5. Verfahren nach Anspruch 3 und 4, wobei der mindestens eine Gewichtungsfaktor für die Änderung der PEL zwischen dem ersten und dem zweiten Bild null ist.

6. Verfahren nach einem der vorhergehenden Ansprüche 3-5, wobei der mindestens eine Gewichtungsfaktor für die Änderung von P zwischen dem ersten und dem zweiten Bild null ist.

7. Verfahren nach einem der vorhergehenden Ansprüche 3-6, wobei der mindestens eine Gewichtungsfaktor für die Änderung der EEL zwischen dem ersten und dem zweiten Bild eins ist.

8. Verfahren nach einem der vorhergehenden Ansprüche, wobei die beiden Bilder den 2., 3. und 4. Mittelhandknochen zeigen.

9. Verfahren nach einem der vorhergehenden Ansprüche, wobei die Lage des Innen- und/oder Außenrands des mindestens einen Knochens auf den mindestens zwei Bildern für eine Mehrzahl von einzelnen Linien bestimmt wird, die sich in der Richtung rechtwinklig zu der Längsrichtung des Knochens erstrecken und an unterschiedlichen Positionen entlang der Längsrichtung des Knochens angeordnet sind, und wobei die Berechnungen des BMDCR auf der Grundlage von Paaren der Lage des Innen- und/oder Außenrands des mindestens einen Knochens auf den mindestens zwei Bildern, die den einzelnen Linien entsprechen, bestimmt wird.

10. Verfahren nach Anspruch 9, wobei die Lage des Innen- und/oder Außenrands des mindestens einen Knochens auf den mindestens zwei Bildern auf der Grundlage der mittleren Lage jeder der einzelnen Linien bestimmt wird.

11. Verfahren nach Anspruch 9, wobei die Lage des Innen- und/oder Außenrands des mindestens einen Knochens auf den mindestens zwei Bildern auf der Grundlage der mittleren Lage jeder von mehr als 10 Linien pro cm des Knochens innerhalb eines vorbestimmten Längsteils des Knochens bestimmt wird.

12. Verfahren nach Anspruch 9, wobei die Lage des Innen- und/oder Außenrands des mindestens einen Knochens auf den mindestens zwei Bildern auf der Grundlage der mittleren Lage jeder von mehr als 40 Linien pro cm des Knochens innerhalb eines vorbestimmten Längsteils des Knochens bestimmt wird.

13. Verfahren nach Anspruch 9, wobei die Lage des Innen- und/oder Außenrands des mindestens einen Knochens auf den mindestens zwei Bildern auf der Grundlage der mittleren Lage jeder von mehr als 60 Linien pro cm des Knochens innerhalb eines vorbestimmten Längsteils des Knochens bestimmt wird.

14. Verfahren nach Anspruch 9, wobei die Lage des Innen- und/oder Außenrands des mindestens einen Knochens auf den mindestens zwei Bildern auf der Grundlage der mittleren Lage jeder von mehr als 80 Linien pro cm des Knochens innerhalb eines vorbestimmten Längsteils des Knochens bestimmt wird.

15. Vorrichtung, umfassend Hardwaremittel und Softwaremittel, die zur Durchführung des Verfahrens nach einem der vorhergehenden Ansprüche beschaffen sind.


**Revendications**

1. Méthode d'identification de la vitesse de variation de la densité minérale osseuse basée sur au moins deux images du même au moins un os prises à deux points différents dans le temps, la méthode comprenant :

    - la détermination de l'emplacement du bord interne et/ou externe dudit au moins un os à partir desdites au moins deux images ;
    - le calcul d'une vitesse de variation de la densité minérale osseuse (BMDCR) en utilisant au moins un facteur de pondération pour la variation entre la première et la deuxième image dudit emplacement du bord interne et/ou externe, dans lequel l'importance dudit au moins un facteur de pondération est choisie en fonction de la cause de la variation de la densité minérale osseuse.

2. Méthode d'identification de la vitesse de variation de la densité minérale osseuse selon la revendication 1, la méthode comprenant également :

    - la détermination d'un ou plusieurs paramètres en rapport avec la densité minérale osseuse,
    - le calcul d'une vitesse de variation de la densité minérale osseuse (BMDCR) en utilisant aussi au moins un facteur de pondération pour la variation entre la première et la deuxième image desdits un ou plusieurs paramètres en rapport avec la densité minérale osseuse, dans lequel l'importance dudit au moins un facteur de pondération est choisie en fonction de la cause de la variation de la densité minérale osseuse.

3. Méthode d'identification de la vitesse de variation de la densité minérale osseuse selon la revendication 1, dans laquelle ledit emplacement du bord interne est l'emplacement du bord endostal (EEL), et ledit emplacement du bord externe est l'emplacement du bord périostal (PEL) dudit au moins un os provenant desdites au moins deux images, et ledit calcul d'une vitesse de variation de la densité minérale osseuse (BMDCR) utilise au moins un facteur de pondération pour la variation entre la première et la deuxième image du PEL qui est inférieur ou égal à l'au moins un facteur de pondération pour la variation entre la première et la deuxième image de l'EEL.

4. Méthode d'identification de la vitesse de variation de la densité minérale osseuse selon la revendication 2, dans laquelle ledit emplacement du bord interne est l'emplacement du bord endostal (EEL), ledit emplacement du bord externe est l'emplacement du bord périostal (PEL) et dans laquelle ledit un ou plusieurs paramètres en rapport avec la densité minérale osseuse est la porosité (P) dudit au moins un os provenant desdites au moins deux images, et ledit calcul d'une vitesse de variation de la densité minérale osseuse (BMDCR) utilise au moins un facteur de pondération pour la variation entre la première et la deuxième image du PEL qui est inférieur ou égal à l'au moins un facteur de pondération pour la variation entre la première et la deuxième image de P et est inférieur à l'au moins un facteur de pondération pour l'EEL.

5.  Méthode selon les revendications 3 et 4, dans laquelle l'au moins un facteur de pondération pour la variation entre la première et la deuxième image du PEL est égal à zéro.

6.  Méthode selon les revendications 3 à 5, dans laquelle l'au moins un facteur de pondération pour la variation entre la première et la deuxième image de P est égal à zéro.

7.  Méthode selon les revendications 3 à 6, dans laquelle l'au moins un facteur de pondération pour la variation entre la première et la deuxième image de l'EEL est égal à un.

8.  Méthode selon l'une quelconque des revendications précédentes, dans laquelle lesdites au moins deux images comprennent les 2e, 3e et 4e os métacarpiens.

9.  Méthode selon l'une quelconque des revendications précédentes, dans laquelle ledit emplacement du bord interne et/ou externe dudit au moins un os provenant desdites au moins deux images est déterminé pour plusieurs lignes individuelles s'étendant dans la direction perpendiculaire à la direction longitudinale de l'os et étant positionnées en différentes positions le long de la direction longitudinale de l'os, et dans laquelle le calcul de la BMDCR est réalisé sur la base de paires dudit emplacement du bord interne et/ou externe dudit au moins un os provenant desdites au moins deux images correspondant aux lignes individuelles.

10. Méthode selon la revendication 9, dans laquelle ledit emplacement du bord interne et/ou externe dudit au moins un os provenant desdites au moins deux images est déterminé sur la base de son emplacement moyen pour chacune des lignes individuelles.

11. Méthode selon la revendication 9, dans laquelle ledit emplacement du bord interne et/ou externe dudit au moins un os provenant desdites au moins deux images est déterminé sur la base de son emplacement moyen pour chaque ligne parmi plus de 10 lignes par cm de l'os dans une partie longitudinale prédéterminée de l'os.

12. Méthode selon la revendication 9, dans laquelle ledit emplacement du bord interne et/ou externe dudit au moins un os provenant desdites au moins deux images est déterminé sur la base de son emplacement moyen pour chaque ligne parmi plus de 40 lignes par cm de l'os dans une partie longitudinale prédéterminée de l'os.

13. Méthode selon la revendication 9, dans laquelle ledit emplacement du bord interne et/ou externe dudit au moins un os provenant desdites au moins deux images est déterminé sur la base de son emplacement moyen pour chaque ligne parmi plus de 60 lignes par cm de l'os dans une partie longitudinale prédéterminée de l'os.

14. Méthode selon la revendication 9, dans laquelle ledit emplacement du bord interne et/ou externe dudit au moins un os provenant desdites au moins deux images est déterminé sur la base de son emplacement moyen pour chaque ligne parmi plus de 80 lignes par cm de l'os dans une partie longitudinale prédéterminée de l'os.

15. Appareil comprenant des moyens matériels et des moyens logiciels adaptés pour exécuter la méthode selon l'une quelconque des revendications précédentes.

Fig. 1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 1046374 B1

- EP 1046374 A